# EUROPEAN PATENT APPLICATION

(11) **EP 3 363 357 A1**
(43) Date of publication of application: **22.08.2018**
(21) Application number: 17156812.4
(22) Date of filing: 20.02.2017
(51) Int. Cl.: A61B 5/05, A61B 5/055, A61B 5/00, G01R 33/28, G01R 33/54, G01N 21/3554, G01N 21/3581

(54) **CONTACTLESS SKIN SURFACE CONDUCTIVITY DETECTOR**

(71) Applicant: Koninklijke Philips N.V., 5656 AE Eindhoven (NL)
(72) Inventor: VAN HELVOORT, Marinus Johannes Adrianus Maria, 5656 AE Eindhoven (NL); VAN DEN BRINK, Johan Samuel, 5656 AE Eindhoven (NL); BENSCHOP, Franciscus Johannes Maria, 5656 AE Eindhoven (NL)
(74) Representative: de Haan, Poul Erik

(57) **Abstract**

A system and a method for detecting an occurrent or forming region of increased electrical conductivity on a human subject's skin surface are disclosed. The system comprises a user interface and a skin surface conductivity detection system. The latter comprises a terahertz transmitter, a terahertz camera for recording terahertz waves reflected off a subject's body, and an image processing unit adapted to execute software code which implements said method. The method comprises receiving image data from the terahertz camera; analyzing the image data for an occurrence of a temporal and / or spatial characteristic featuring an electrical conductivity which is related to a predefined threshold at which a thermal burn of the subject's skin surface at the region is avoided during exposure to an RF field of a magnetic resonance imaging system for imaging the subject; and sending information regarding the analysis to the user interface.

## Description

### FIELD OF THE INVENTION

The present invention relates to terahertz imaging in the context of a medical magnetic resonance imaging system.

### BACKGROUND OF THE INVENTION

Magnetic resonance imaging (MRI) has become an indispensable tool in modern medicine through the last decades. Its strong and versatile use in internal medicine notwithstanding, there is a risk that injuries to a human subject may occur during magnetic resonance imaging. Among these injuries are skin burns which may occur if a region of increased electrical conductivity forms on the subject's skin and an electric current gets induced within that region by resonant coupling of a radio-frequency (RF) field which is required for the normal functioning of the MRI system. The severity of MRI caused burns may range up to the third degree, which requires an additional medical treatment of the subject. Numerous case reports in scientific publications, such as the recently published "MRI-Induced Second and Third Degree Burns to Hands Bilaterally: A Case Report" by Viscuse PV et al., Ann. Hematol. Oncol., Volume 2, Issue 7, 2015.

The most important reasons for MRI-induced skin burns which are known today are metallic microfibers in clothing, crossing galvanic cables or skin-to-skin contact. These features have in common that they may carry an electric current induced by the subsequent RF pulses generated by the MRI system if their geometry allows for the formation of a closed loop which is resonant to the frequency of the RF pulses. Heat is then generated by the electrical resistance of the resonance conductive structure on or in contact with the subject's skin. Despite thorough pre-examination routines, the formation of resonant current loops on the subject's skin cannot be avoided in some cases because the peculiarities responsible for their formation, such as moisture forming on the subject's skin during an MRI scan or microscopic metallic fibers deposited on the skin from the subject's clothing, are often invisible.

### SUMMARY OF THE INVENTION

In a first aspect, the invention relates to a system for detecting a temporal and / or spatial characteristic of increased electrical conductivity on a human subject's skin surface, wherein the system comprises:
- a user interface, and
- a skin surface conductivity detection system, comprising:
   - a terahertz transmitter adapted for emitting terahertz waves in the subject's direction,
   - a terahertz camera adapted for recording terahertz image data from a portion of the terahertz waves reflected off the subject's skin, and
   - an image processing unit communicatively coupled to the terahertz camera, the image processing unit comprising a processor and memory, the memory comprising software code which, upon execution by the processor, causes the image processing unit to:
      - receive the image data from the terahertz camera,
      - analyze the image data for an occurrence of a temporal and / or spatial characteristic of electrical conductivity, the analysis comprising relating the characteristic of electrical conductivity to a predefined threshold, the threshold representing a magnitude of an electric current in the human subject's body at which a thermal burn of the subject's skin surface is avoided during exposure to an RF field of a magnetic resonance imaging system for imaging the subject, and
      - send information regarding the occurrence to the user interface.

Thus, mostly invisible features responsible for increased electrical conductivity on the skin surface may be visualized by means of terahertz imaging. Electrically conducting objects appear bright in terahertz images because terahertz waves become reflected by them. Additionally, terahertz waves become strongly absorbed by water, which may be exploited as moist skin areas appear darker on absorption mode terahertz images recorded with increased sensitivity. The described system may therefore serve as an appropriate technology for detecting areas on a human subject's skin surface which fulfill the requirements for causing skin burns by unintentionally induced resonant electric currents on the skin surface.

The system may allow for detecting a temporal and / or spatial characteristic of increased electrical conductivity with respect to a normal level of electrical conductivity usually measured on dry skin. This may be achieved by accordingly calibrating, or biasing, the sensitivity of the terahertz camera with a respective threshold level. Alternatively, the camera may be operated unbiased and a biasing may be applied to the recorded terahertz image data subsequently by means of software. This way, only skin surface features will be recorded which produce a terahertz signal which exceeds the threshold level. Another alternative may be to detect the variation in the terahertz signal characteristic, as far as it represents conductivity, then apply a regression analysis, and show the change over time or apply a threshold to this rate of change in characteristic. All mentioned alternatives may be applied to reflectivity measurements as well as absorptivity measurements on an inverted scale.

The system may be equipped with a magnetic resonance imaging (MRI) system. An MRI system may be any 2D, 3D, and/or 4D imaging system which is capable of visualizing inner tissue by time and spatially resolved observation of the decay of nuclear spin magnetization within said tissue. In particular, MRI systems include coils for generating gradient magnetic fields, coils driven by a pulse generator for generating short pulses of RF waves or fields for exciting nuclear spins in the body of the subject to be imaged, coils for receiving in response to the short pulses RF signals from the magnetized nuclear spins present in the subject's tissue, and/or further electric and/or electronic equipment or circuitry as necessary for decoding the RF responses received from the subject's body into a visual image representing a two-dimensional slice of said inner tissue. MRI systems are commercially available and widespread within medical, especially clinical, environments.

The system further includes a user interface for controlling at least the skin surface conductivity detection system. For example, the user interface includes input and output devices, where the input devices include, but are not limited to, a keyboard, a mouse, buttons, or a speech control unit; whereas the output devices may include, but are not limited to, a visual monitor, screen, or display, manufactured in any known technology such as electron ray tubes, liquid crystal displays, or matrices of light emitting diodes, but also visual indicators such as single LEDs, or lamps, furthermore loudspeakers etc. Components of the user interface may be integrated in one or more devices of the skin surface conductivity detection system or the magnetic resonance imaging system.

The skin surface conductivity detection system is based on active imaging technology, i.e., it includes a terahertz transmitter and a terahertz camera. The terahertz range of the electromagnetic spectrum is roughly coincident with wavelengths between 0.1 and 1 mm. The system may include one or more transmitters, which may be operated in continuous wave or pulse mode or may be switched on and off on demand for every single image recorded by the camera. In the past decade, terahertz transmitters, receivers, and imaging devices have become commercially available as solid state devices working at room temperature. First application scenarios include security scanners at public places such as airports and monitoring units to detect irregularities in industrial production lines. The same technology may be employed with the present invention, as well as dedicated devices fit to the particular purpose of skin surface imaging. It must, however, be noted that specific measures may have to be applied in addition if the system is used in direct vicinity or integrated in an MRI system to ensure compatibility of the terahertz and MRI equipment, such as electromagnetic shielding of the electronics, selection of non-magnetic components, and / or communication over optical fiber.

A terahertz transmitter generally comprises a primary oscillator and an antenna for coupling out its output as a space wave. The primary oscillator may be a local device forming a unit with the antenna, but also a remote device whose signal is transferred to the antenna through a coaxial cable and/or a waveguide. Especially for a remote oscillator, it may be useful to operate the primary oscillator at a microwave frequency where sufficient output power is available, and multiplying the frequency of the microwave signal in the vicinity of the antenna using a diode or a similar solid state device with a non-linear I-V characteristic. Other miniaturized sources of terahertz waves include solid-state quantum cascade lasers or photomixing sources, where there terahertz radiation is generated as the difference frequency of two higher-frequent sources e.g. from the optical or infrared range. An antenna producing a directed terahertz beam is preferable for the purposes of the present invention. The terahertz beam generated by the terahertz transmitter may be monochromatic or a broadband signal.

The terahertz transmitter emits terahertz waves in the subject's direction, where they get absorbed and scattered into the direction of the terahertz camera. The terahertz camera basically comprises an array of microbolometers or diodes such as Schottky diodes which are read out by suitable electronics to produce a pixeled image of the terahertz waves received from the subject's skin surface. The terahertz camera is a solid state room temperature device of commercial origin or in a bespoke implementation. Cameras and transmitters may be available as a matching pair. It usually includes further electronics such as low noise amplifiers or filtering circuitry, as well as optical elements in front of the pixel array, such as filtering plates or lenses, to match the incoming terahertz beam to the detecting plane.

It is understood that one or more cameras may be present in the skin surface conductivity detection system as necessary to provide the desired coverage of the subject's skin surface. Particularly, it is possible to use a specific coverage instead of imaging the full body. Preferred locations of the multiple detectors can be determined based on characteristics of the subject and, if present in the system, the MRI system. Examples of such preferred locations comprise the end ring of the MR system body coil, the outer shoulder area, the curvature from neck to shoulders, hands and thighs, and the inner thighs of the subject.

Both the antenna opening of the terahertz transmitter and the objective aperture of the terahertz camera may be covered by a window plate manufactured from a material such as polyethylene, Teflon or mica, which is transmissive to the terahertz signal emitted by the terahertz transmitter. Cover windows protect the interior of the terahertz equipment from contaminants such as dust particles or undesirable variations such as changes of air humidity or temperature. One or more of the optical elements mentioned above may be integrated in the terahertz windows to provide, for example, an improved beam guide to match the particular optical conditions encountered in the skin surface imaging situations described herein. A thick lens disposed on the transmitter antenna to focus or defocus the terahertz beam or a Fresnel lens disposed on the objective of the camera to focus the incoming beam with a minimum of attenuation shall be listed here as two non-exhaustive examples.

Furthermore, the skin surface conductivity detection system comprises an image processing unit. This unit may be provided as a standalone device, but it may also be integrated into one or more devices of the magnetic resonance imaging system. The image processing unit comprises at least a processor and memory for analyzing the image data received from the terahertz camera. It may comprise further suitable components such as a communication controller together with communication interfaces for exchanging information with the terahertz camera and/or the terahertz transmitter, as well as the user interface and/or the optional magnetic resonance imaging system, depending on the degree of integration; the primary oscillator for the terahertz transmitter; control circuitry for controlling the transmitter and/or the camera; mathematical and/or graphical co-processors to improve analysis performance; etc. The communication may be performed through a wireless or a wired connection, such as the parallel or serial bus, e.g. USB, or Ethernet. The mentioned components of the image processing unit may be partly or fully integrated in the optional MRI system, whereas the functions provided by the image processing unit may be integrated in MRI control software of the MRI system as applicable to the extent described herein.

The image data received by the image processing unit from the terahertz camera comprises a digital two-dimensional representation of a part of the subject's skin within at least the frequency range emitted by the terahertz transmitter. The image processing unit is adapted for analyzing this image data for an occurrence of a temporal and / or spatial characteristic of increased electrical conductivity due to e.g. the presence of moisture or a tattoo on the subject's skin. For this purpose, the memory of the image processing unit comprises software code with instructions for verifying a set of criteria which must be fulfilled for successful identification of such characteristic. In an example, the characteristic may be a region on the subject's skin and the image data may be required to contain pixels of a reflection or absorption intensity or, if a temporal characteristic is taken into account, intensity change exceeding a threshold level representing the natural electrical surface conductivity of dry skin. The size of such region does not necessarily play a role: If skin gets wet, there is a chance for a low impedance for high frequency currents between two neighboring body parts. These currents are generated in the large area of the body, but may cause burns only in a small contact area or wet area of the skin.

There are, however, situations where the pixels exceeding the threshold may be required to have a size or geometry which allows for resonant coupling of an RF wave generated by the magnetic resonance imaging system. This may be the case because a large wet surface may change the conversion of RF energy to beyond specific absorption rate (SAR) values which have been simulated for normal body models. The size may also play a role if the system is used to identify a tattoo. It may thus be beneficial to check whether a current loop of a resonant diameter fits into an identified continuous region of pixels representing increased values of electric skin surface conductivity.

The image processing unit relates, or compares, the image data to a magnitude of an electric current caused in the human subject's body by an RF field of a magnetic resonance imaging system. For this purpose, a processor of the image processing unit may calculate an electric current which is to be expected under the given conditions at the position on the subject's skin being currently analyzed. Conditions taken into account may include a local intensity of the RF field, the frequency of the RF field, personal or medical parameters such as age or skin type of the subject, a theoretical or empirical model of the subject's skin conductivity which may or may not be representing the subject's personal or medical parameters, body contour data representing the subject, etc. Such conditions may, for instance, be comprised by the software code, received as a user input into the user interface, and / or retrieved from an appropriate database. In a particular example, a user of the system may use the user interface to select one of the two typical RF frequencies 64 MHz and 128 MHz from a setup menu.

The verification of said criteria can be broken down to basic calculation operations which can be performed by a standard graphics processing unit (GPU) or any other processor programmed to work through suitable graphics analysis routines. Particularly, a segmentation algorithm may be used to identify regions in the image data, or a difference image calculated from two different sets of image data acquired with the terahertz camera for the same subject, in which exceed a threshold for electrical conductivity, current magnitude, or current density is exceeded. As image acquisition conditions or image quality may vary for changing subjects, detection efficiency may be increased by using algorithms which provide variable-scale or multi-scale image processing. Of course, it is alternatively possible that e.g. a contour of a region to be analyzed or monitored may be input by a user of the system via the user interface.

The image processing unit is also configured to send information regarding the analysis to the user interface. In the simplest case, said information may comprise the image data received from the terahertz camera. A display of the user interface may then show a visual representation of the terahertz image data to a person such as a medical examiner who is operating an MRI system. Detected or presumable temporal and / or spatial characteristics of electrical conductivity may be highlighted in the representation, e.g. by displaying such regions in different colors, drawing a frame around pixels representing such characteristic, and / or flashing such pixels to draw a user's attention to the characteristic.

The information may also comprise a text message informing the user about the result of the most recent image data analysis. In case of a positive detection of a temporal and / or spatial characteristic of electrical conductivity which might cause skin burns to the subject, this text message may comprise an alert message. The information may also comprise an acoustic or optical signal or alternatively a control signal in order to cause, for example, a loudspeaker or a lamp of the user interface to issue an acoustic or an optical signal, respectively. It is also possible that the information sent to the user interface comprises MRI control signals such as a stop, interrupt, or interlock signal in the case that a potentially harmful characteristic has been detected in the image data.

Advantageously, the system may enable a user to detect regions and / or objects which would cause skin burns to the subject if they remained on or in contact with the subject's skin. Such objects can be hidden from view because they are located under the subject's clothing; they may be transparent to the eye such as moisture or sweat present on the skin; they may be too small to be identified with the naked eye, as is the case for metallic microfibers which are increasingly used in sports underwear; or they may be too difficult to identify for a normal MRI operator who is not specially trained for identifying e.g. areas of skin-to-skin contact with increased skin moisture or cables touching the subject more than once. The present invention provides means to visualize and detect such features safely without touching the subject's body or even trying to measure the subject's electrical skin surface conductivity directly.

The use of terahertz waves may be advantageous over other bands of the electromagnetic spectrum because it has a unique combination of features: non-metallic objects, particularly most kinds of clothes, appear transparent in the terahertz range, enabling the possibility to observe a subject's skin with no need to remove their clothing. Terahertz wavelengths are short enough to enable imaging with sufficiently high resolution to locate the position of a suspect area with sufficient precision. Furthermore, it is possible to identify materials that possess a unique spectral fingerprint in the terahertz range, which may enable an automated distinction of materials to further delimit the kind of feature which is causing increased electric conductivity on the subject's skin. This may be of particular relevance in detecting safe and unsafe pigments in (large) tattoos.

In an embodiment, the software code, upon execution by the processor, further causes the image processing unit to provide an interlock signal which is adapted to cause a magnetic resonance imaging system to abort the imaging. During MR imaging, the subject's skin may be subject to changes. The subject may feel warm and start sweating, or slight changes in body position can spontaneously create areas of close skin-to-skin contact with increasing moisture. If such a region of spontaneously increased conductivity is created, aborting the imaging may protect a subject from suffering skin burns although nothing might have been found during a screening in preparation of the imaging.

In another embodiment, the system further comprises a magnetic resonance imaging system which comprises an RF coil or antenna for generating the RF signal, wherein the terahertz camera is integrated in the RF coil or antenna. There are other embodiments where the terahertz camera is one out of a plurality of terahertz cameras integrated in the RF coil or antenna. This may enable a continuous or intermittent monitoring of a subject during an MRI scan.

According to an embodiment, the analyzing of the image data further comprises comparing the image data with previously recorded terahertz image data, wherein the threshold represents a temporal characteristic, or difference, of electrical conductivity with respect to the previously recorded terahertz image data. This may be helpful for noticing changes of the subject's skin which could cause or lead to skin burns due to RF induction. According to another embodiment, the software code, upon execution by the processor, further causes the image processing unit to record the terahertz image data during imaging of the subject in the magnetic resonance imaging system. Detecting changes of electrical conductivity may be beneficially used for monitoring a subject during an MRI scan. This may allow a person operating an MRI system to keep track of increasing conductivity or humidity of the subject's skin and react early enough so that skin burns can be prevented or a running scan does not have to be aborted. The operator may also receive a notification e.g. if a detected change exceeds a pre-determined threshold. The comparison may be performed between subsequent terahertz images or also against earlier images to detect slow conductivity changes.

According to one embodiment, the magnetic resonance imaging system further comprises a magnetic material detector (gate or pillar) to be passed by the subject before performing the imaging, with the terahertz camera being integrated in the gate. This embodiment may help to identify objects which would cause skin burns if not removed before starting the imaging. It may also help detect that the subject is wearing clothes equipped with metallic microfibers, a fact even the subject may be unaware of.

In a further embodiment, the terahertz transmitter, the terahertz camera and the user interface are comprised in a handheld device. A handheld device may be beneficial for screening a subject more flexibly before starting the imaging, especially to detect features which are hidden under the subject's clothes such as tattoos, piercings or jewelry in places which are not related to the body region to be imaged with an MRI system. It may also allow for in-situ inspection of an area e.g. where the subject started to feel a pain for no obvious reason.

According to embodiments, the information comprises the image data and / or an alert message. It may be beneficial to keep a user of the system up to date about changes to the subject's skin, especially electrically conductive areas which may form e.g. during an MRI scan, but do not fulfill all criteria for resonant coupling of the RF field yet. This way, an MRI operator may react to forming suspect areas even before an alarm is raised. If an electrically conductive characteristic is positively detected, the user may be informed by an alert message such as a text message, a flashing lamp or part of a display, an acoustic signal etc. to be able to react on the possible incident immediately.

The invention may also allow for inspecting the geometry of the characteristic for being resonant in a manner that heat is generated on the subject's skin by induction of an electrical current, a quantity of the heat exceeding a threshold value for injury of the subject. This may have the advantage that the conditions responsible for RF-induced skin burns are assessed more precisely. In particular, the skin surface conductivity system may have information about the RF frequency actually used by a particular MRI system. This information may serve as a basis for calculating the correct size of a resonant geometry, thus reducing the occurrence of false positive detections.

Furthermore, the image processing unit may include the intensity information provided by the image into a calculation to estimate the amount of heat which is generated in the suspect resonant structure. This may give an MRI operator a more precise indication of the actual risk of skin burns to which the subject is currently exposed. The system may e.g. include in its information sent to the user interface that there is a resonant structure, but the risk of suffering skin burn is negligible because the amount of heat generated is too low. Such low-risk areas may then e.g. be shown in the terahertz image data on a display of the user interface with a different signaling color than areas where the subject is at immediate risk of getting burned.

In an alternative embodiment, the electrical conductivity is the result of skin moisture of the subject and the terahertz camera is configured for being sensitive to absorption of the terahertz waves caused by the skin moisture of the subject.

Water is a strong absorber for terahertz waves and will appear with a reduced intensity, i.e. in absorption, in an image generated by the terahertz camera. If the skin surface conductivity detection system was solely configured to measure scattered terahertz waves, it may not notice the formation of an electrically conductive area due to skin moisture because its terahertz intensity is below the reflectivity threshold level. Hence, if the camera or the analysis software is configured to detect absorption intensity, e.g. by being calibrated against a particular absorption threshold value for dry skin and applying an inverted scale to the image data, the system may be able to also detect potentially resonant absorbing areas or features on the subject's skin. This may be beneficial because the variety of detectable causes for MRI-induced skin burns is increased.

It may be beneficial, too, to switch the operating mode of the camera and / or software between detection in scattering and absorption modes to make full use of the detection capabilities offered by terahertz imaging.

According to an embodiment, the threshold represents an absolute value of an electrical conductivity. Such value may, for instance, the result of a calibration performed with the subject in a condition where no risk of RF burns could be detected; a theoretical or empirical literature value for human skin surface conductivity under certain standard conditions; and / or a value retrieved from a database with empirical values for different skin types, subject categories, or present features such as wound tissue, different types of tattoo ink, etc. This may provide an advantageous effect by taking into account specific conditions of a particular subject's skin.

A further aspect of the invention relates to a method for detecting a temporal and / or spatial characteristic of increased electrical conductivity on a human subject's skin surface, using:
- a user interface, and
- a skin surface conductivity detection system, comprising:
- a terahertz transmitter,
- a terahertz camera, and
- an image processing unit communicatively coupled to the terahertz camera,
wherein the method comprises a conductivity detection procedure comprising:
- emitting terahertz waves in the subject's direction using the terahertz transmitter,
- recording terahertz image data from a portion of the terahertz waves reflected off the subject's skin,
- receiving the image data from the terahertz camera using the image processing unit,
- analyze the image data for an occurrence of a temporal and / or spatial characteristic of electrical conductivity, the analysis comprising relating the characteristic of electrical conductivity to a predefined threshold, the threshold representing a magnitude of an electric current in the human subject's body at which a thermal burn of the subject's skin surface is avoided during exposure to an RF field of a magnetic resonance imaging system for imaging the subject, and
- send information regarding the analysis to the user interface.

The method may further comprise performing the imaging of the subject using the magnetic resonance imaging system, the conductivity detection procedure being repeatedly performed during the imaging in pre-determined time intervals until the imaging is finished. This may allow for a continuous or intermittent monitoring of the subject for changes in skin conductivity during an MRI scan.

Optionally, the method may further comprise performing the imaging of the subject using the magnetic resonance imaging system only in case that the analyzing has resulted in a non-detection of the occurrence. This may prevent an MRI operator from starting an MRI scan with the subject if an electrically conducting characteristic is found in the subject's skin.

In a further aspect, the invention relates to a computer program product for detecting a temporal and / or spatial characteristic of increased electrical conductivity on a human subject's skin surface, the computer program product comprising a computer-readable storage medium having program instructions embodied therewith, the program instructions being executable by a processor to cause the processor to execute the method as described above.

It is understood that the various embodiments may be combined as long as they are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory

(RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage may be any volatile or non-volatile computer-readable storage medium.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the C programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) display, Electroluminescent display (ELD), Plasma display panel (PDP), Liquid crystal display (LCD), Organic light-emitting diode display (OLED), a projector, and Head-mounted display.

Magnetic Resonance (MR) data is defined herein as being the recorded measurements of radio frequency signals emitted by atomic spins using the antenna of a magnetic resonance apparatus during a magnetic resonance imaging scan. Magnetic resonance data is an example of medical imaging data. A Magnetic Resonance (MR) image is defined herein as being the reconstructed two or three dimensional visualization of anatomic data contained within the magnetic resonance imaging data.

The computer program product may be implemented as a separate piece of software, but it may also be part of a software suite for a different purpose, particularly a control software for an MRI system. The software may be executed on a dedicated computer for controlling the terahertz transmitter and the terahertz camera, or on a computer related to an MRI system, in particular a computer dedicated to controlling a magnetic resonance imaging session. The computer program product may be beneficial as it enables the user to perform any one of the methods according to the second aspect of the invention using a computer.

### BRIEF DESCRIPTION OF THE DRAWINGS

In the following preferred embodiments of the invention will be described, by way of example only, and with reference to the drawings in which:
Fig. 1 shows an example of a skin surface conductivity detection system in the context of a magnetic resonance imaging system,
Fig. 2 illustrates an integration example of a skin surface conductivity detection system into an auxiliary instruments segment of an RF coil,
Fig. 3 illustrates an alternative integration example of a skin surface conductivity detection system integrated into an auxiliary instruments segment of an RF coil, and
Fig. 4 shows a front view (A) and a back view (B) of a skin surface conductivity detection system implemented as a handheld terahertz imager.

### DETAILED DESCRIPTION OF EMBODIMENTS

A human subject undergoing a magnetic resonance imaging (MRI) scan has a risk of getting injured by resonant electric currents induced into skin parts exhibiting an increased electrical conductivity. The electric currents may also be induced in metallic micro fibers in clothing that is in contact with the subject's skin. The electric currents may be induced in a certain region of electrical conductivity due to a resonance of the geometry of conductivity to the RF field employed during the MRI scan. Such conducting skin areas or features causing increased skin surface conductivity are often invisible. Hence, there is a need for a system enabling visualization of electrically conducting skin areas or objects, and also verification whether a potentially harmful conductivity feature fulfills conditions for resonant response to an RF field generated by the MRI system.

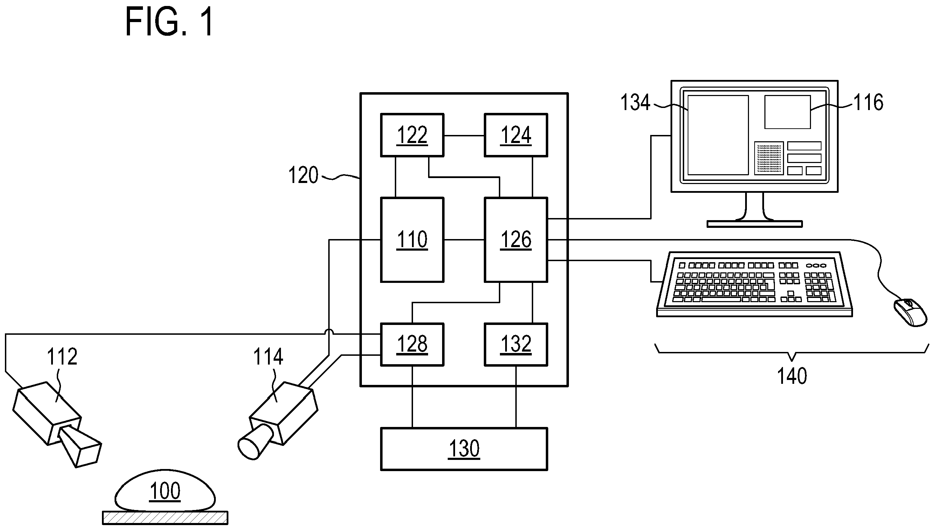

An example of a system for detecting a temporal and / or spatial characteristic of increased electrical conductivity on a subject's skin surface is shown in Fig. 1. A terahertz transmitter 112 and a terahertz camera 114 are arranged in front of a subject 100 such that the camera 114 receives terahertz waves generated by the transmitter 112 and reflected off the subject's body. This arrangement may in principle represent any situation where the subject 100 is observed for occurring or changing regions of electrical conductivity on the skin surface. Non-exhaustive examples include the subject 100 lying on an examination couch or table situated inside a coil of an MRI system, the transmitter 112 and the camera 114 being integrated in this coil; the transmitter 112 and camera 114 being part of a handheld device 400 for examining the subject's skin surface for electrically conductive objects that may be hidden under the subject's clothes, such as metallic piercings, needles, rings or other pieces of jewelry, metallic fibers contained within the subject's clothing or deposited on the subject's skin from clothes having been worn before the examination, moist skin surface areas, tattoos which may be visible in the terahertz range due to variations in electrical conductivity caused by tattoo ink, or areas of skin-to-skin contact which may be electrically conductive due to sweat; or also an arrangement where camera 114 and transmitter 112 are installed in a wall-mounted apparatus which is used to check the subject 100 for paramagnetic objects in preparation of an MRI scan.

Camera 114 and transmitter 112 are shown here as autonomous devices which are merely fed with electrical power by a power controller 128 which is part of the control unit 120 of the magnetic resonance imaging system 130. The hardware and software components apart from transmitter 112 and camera 114 are fully integrated into the MRI system 130. Resources of the control unit 120, such as processor 122, memory 124 and communication controllers, are shared between the skin surface conductivity detection system and the MRI control unit 120. The central part of the control unit 120 is the user interface controller 126, which is connected to the processor 122, the memory 124, the image processing unit 110, the power controller 128 and the MRI controller 132. It comprises the mentioned communication controllers for exchanging information between the user interface 140, the MRI system 130 and the terahertz devices, as suitable sets of input/output controllers, a graphics controller, and supplemental electronic circuitry needed for a full functioning of the MRI system 130. Control commands issued by the user interface controller 126 for the MRI system 130 are translated into machine level commands by the separate MRI controller 132. Electrical power is supplied to the MRI system 130 and the terahertz devices by the power controller 128. The magnetic resonance imaging system 130 comprises one or more coils for generating a gradient magnetic field and an RF field at the subject's location, and for detecting magnetization decay signals from the magnetized nuclei in the subject's tissue to be imaged; and further electronic circuitry involving, for example, pre-amplifiers, filters, compressors, etc.

The software level integration becomes noticeable at the user interface 140, which comprises a computer screen or monitor, a keyboard and a mouse. The MRI control software running on the control unit 120 displays the window on the screen where the most recent terahertz image recorded by the terahertz camera 114 is shown next to a large image displaying the recently recorded slice of MRI data 134 generated by the MRI system 130. The window includes further information and control objects, allowing a user to control both the MRI system 130 and the skin surface conductivity detection system.

During operation of the system, terahertz image data 116 generated by the terahertz camera 114 is received by the image processing unit 110, which may comprise a dedicated graphics processing unit GPU and graphics memory for performing the analysis of the image data 116, as well as a communication interface for exchanging information at least with the terahertz camera 114. Each image received from the terahertz camera 114 is stored in the graphics memory of the image processing unit 110 and/or the memory 124 of the control unit 120. The GPU and/or the processor 122 of the control unit 120 perform the necessary analysis steps for detecting an occurrence or change of a region of electrical conductivity on the subject's skin surface.

An occurrence may be detected by verifying that a number of pixels has intensity values exceeding a threshold value associated with the natural electrical conductivity of dry skin, and that they form a contiguous region such that an electrical current may be generated by resonant coupling of the RF field generated by the MRI system 130 to the electrically conducting area. The RF-induced current may be generated as a loop which fits into in a large contiguous conducting region, but it may also be present in a low-conductivity region while burning the subject's skin only in a small area of increased conductivity acting like a short circuit for the loop. Therefore, the size of a conducting region should not be a necessary criterion for detecting characteristics with a risk for RF-induced skin burns to the subject.

The image processing unit 110 sends information regarding the analysis to the user interface 140. This information may include the terahertz image data 116 recorded by the terahertz camera 114, status information describing a result of the analysis, alert messages and/or signals in the case that the detection was successful, and/or control signals for the MRI system 130, including a signal for aborting a current MRI imaging scan in the case that an electrical conducting characteristic was detected on the subject's skin. As the image processing unit 110 and the components of the user interface 140 are connected to the user interface controller 126, it is understood that the MRI control signals, which may be contained within said information, must be intercepted by the user interface controller 126 to convey the control signal to the MRI system 130 and/or the MRI controller 132 before it is forwarded to the user interface 140.

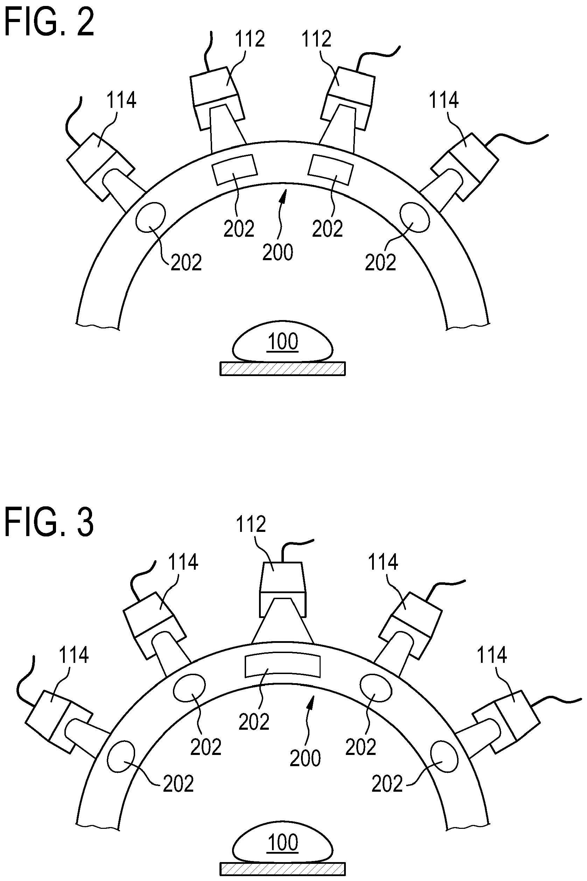

Now turning to Fig. 2, an auxiliary instrument segment 200 of an RF coil is shown, where the subject 100 is lying on an examination table centered with the coil. The auxiliary instrument segment 200 has two terahertz transmitters 112 and two terahertz cameras 114 of the skin surface conductivity detection system embedded inside a gap of the coil and located behind an outer wall of the coil housing. The two cameras 114 are disposed near the zenith of the coil opening, radiating terahertz waves into the subject's direction. The two cameras 114 are located at about 45° distance from the zenith, where the intensity of terahertz waves scattered off the subject's skin surface is greatest. The arrangement shown may allow for a more complete coverage of the subject's skin surface by using more than one terahertz camera 114, and a brighter and more homogenous illumination of the subject 100 is achieved by the use of more than one terahertz transmitter 112. Furthermore, it may be advisable to use more than one terahertz camera 114 in order to compensate for the lower optical resolution of terahertz cameras 114, which is a consequence of the substantially lower wavelength of the terahertz range compared to infrared or optical cameras.

Coverage and resolution of the subject's skin may be increased further by adding more cameras 114 to the auxiliary instrument segment 200 in the same vein. This is illustrated by Fig. 3, where one terahertz transmitter 112 is located in the zenith of the RF coil opening, and two terahertz cameras 114 are located on each side of the coil, resulting in an even more complete coverage of the subject's skin surface by a large terahertz image which is composed of four single images. As only one central transmitter 112 is used here, this setup necessitates a more powerful terahertz transmitter 112 and, optionally, a diffuser placed on the aperture of the transmitter 112 antenna to enable a more homogeneous illumination of the subject 100 by increasing the brightness at the subject's sides. It is, however, also possible here to use more than one less powerful transmitter 112 if enough space is available in the auxiliary instrument segment 200.

The terahertz cameras 114 and transmitters 112 shown in Figs. 2 and 3 are covered with terahertz windows 202 which are transparent in the terahertz range and allow, for example, for placing optical elements in front of the respective opening apertures of the terahertz devices. Terahertz windows 202 may also have the beneficial effect of protecting the interior of the terahertz devices from foreign matter such as dust and disturbing influences like variations of air humidity. They may also provide a more comfortable experience to the subject 100 as there are no openings present in the coil housing. It is even possible to integrate the terahertz windows 202 with the coil housing, i.e., to manufacture parts of the coil housing as terahertz windows 202 such that all of the terahertz devices can be hidden behind a smooth, featureless surface of the coil housing. Terahertz transmissive windows and optical elements can be easily manufactured from inexpensive materials such as polyethylene, which may also be used for parts or the whole of the remaining coil housing.

Adequate protection against electromagnetic interference (EMI) of the sensitive terahertz equipment may also be provided by a suitable additional configuration of the terahertz windows. Alternatively, the MRI and the THz camera can be operated alternately, i.e. the terahertz output is muted during the MRI acquisition process and vice versa. As, for instance, the development of sweat is a relatively slow process, sampling the THz signals on a rate of 5 - 0.1 Hz may be sufficient.

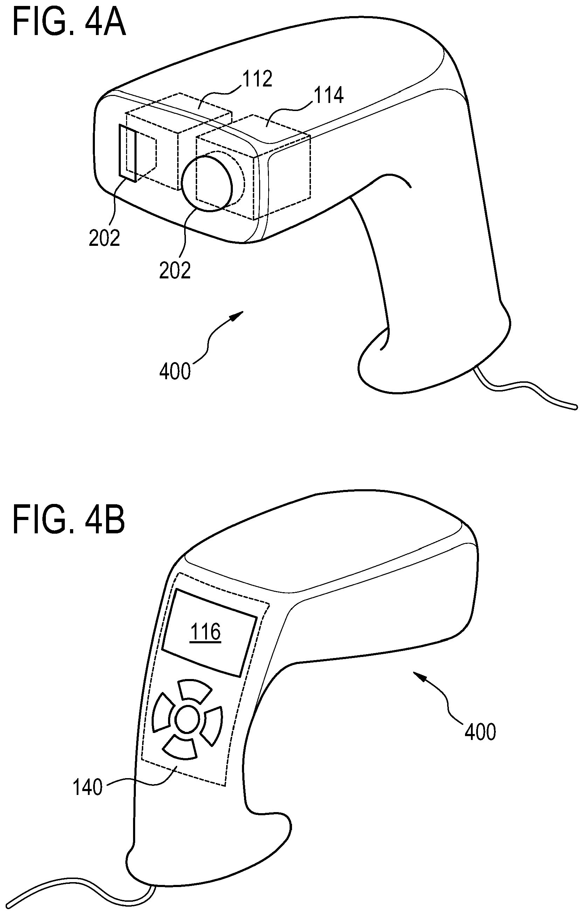

A different exemplary embodiment of a skin surface conductivity detection system is shown in Fig. 4. Here, the terahertz transmitter 112 and the terahertz camera 114 are integrated in a handheld device 400. It consists of an angled housing with the terahertz devices built into an upper part of the housing, the user interface 140 located at the back of the housing, and an ergonomic handle being formed from the lower part of the housing. Once more, transmitter 112 and camera 114 are autonomous devices which do not need to be supplied with microwave power from a remote primary oscillator.

The image processing unit 110 may be integrated with the user interface electronics. The user interface 140 consists of a display and control buttons which allow for user interactions such as menu-driven handling. The handheld device 400 is supplied with electrical power through a cable and does not require further cables or communication interfaces. It can therefore be used for flexible in-situ inspection of the subject 100 for electrically conducting skin surface regions. The handheld device 400 may also be driven by a battery pack which meets the power requirements of the terahertz equipment and the image processing circuitry. The image processing unit 110 can be configured to send the terahertz image data 116 to the display at a faster repetition rate compared to, e.g. the monitoring of the subject 100 during an MRI scan, where it is not necessary to update the terahertz image more often than, e.g. once per minute. In contrast, it may perform a real time update of the display which, e.g. ten images per second, allowing for a more reliable and comfortable inspection of the subject 100.

Once more, the objective apertures of the terahertz devices are sealed with terahertz windows 202, as can be seen from Fig. 4A. The user interface 140, shown in Fig. 4B, may comprise further elements such as a loudspeaker for giving the user an acoustic signal if the occurrence or formation of a potentially resonant electrically conductive region was found on the subject's skin surface, or adjusting wheels for changing, e.g. brightness and contrast of the display image. The image processing unit 110 may also be configured to change color for pixels which fulfill one or more detection criteria for resonant characteristics of electrical conductivity on the subject's skin surface. As an example, regions in the terahertz image which fulfill some criteria, but not all of them, may be shown in a first warning color such as yellow, while pixels of regions where all detection criteria have been successfully verified by the analysis may be shown in a second warning color such as red. This way the speed and reliability of the in-situ inspection may be further increased.

### LIST OF REFERENCE NUMERALS

- 100: human subject
- 110: image processing unit
- 112: terahertz transmitter
- 114: terahertz camera
- 116: terahertz image data
- 120: control unit
- 122: processor
- 124: memory
- 126: user interface controller
- 128: power controller
- 130: magnetic resonance imaging system
- 132: MRI controller
- 134: magnetic resonance image data
- 140: user interface
- 200: auxiliary instruments segment
- 202: terahertz window
- 400: handheld terahertz imager

## Claims

1. A system for detecting a temporal and / or spatial characteristic of increased electrical conductivity on a human subject's skin surface, the system comprising:
- a user interface (140), and
- a skin surface conductivity detection system, comprising:
- a terahertz transmitter (112) adapted for emitting terahertz waves in the subject's direction,
- a terahertz camera (114) adapted for recording terahertz image data (116) from a portion of the terahertz waves reflected off the subject's skin, and
- an image processing unit (110) communicatively coupled to the terahertz camera (114), the image processing unit (110) comprising a processor and memory, the memory comprising software code which, upon execution by the processor, causes the image processing unit (110) to:
- receive the image data (116) from the terahertz camera (114),
- analyze the image data (116) for an occurrence of a temporal and / or spatial characteristic of electrical conductivity, the analysis comprising relating the characteristic of electrical conductivity to a predefined threshold, the threshold representing a magnitude of an electric current in the human subject's body at which a thermal burn of the subject's skin surface is avoided during exposure to an RF field of a magnetic resonance imaging system (130) for imaging the subject (100), and
- send information regarding the analysis to the user interface (140).

2. The system of claim 1, the software code, upon execution by the processor, further causing the image processing unit (110) to provide an interlock signal, the interlock signal being adapted to cause the magnetic resonance imaging system (130) to abort the imaging.

3. The system of any of the previous claims, the information comprising the image data (116).

4. The system of any of the previous claims, the information comprising an alert message.

5. The system of any of the previous claims, the electrical conductivity being the result of skin moisture of the subject (100), the terahertz camera (114) being configured for being sensitive to absorption of the terahertz waves caused by the skin moisture of the subject (100).

6. The system of any of the previous claims, the threshold representing an absolute value of an electrical conductivity.

7. The system of any of the previous claims, further comprising the magnetic resonance imaging system (130), the magnetic resonance imaging system (130) comprising an RF coil for generating the RF signal, the terahertz camera (114) being integrated in the RF coil.

8. The system of claim 7, the terahertz camera (114) being one out of a plurality of terahertz cameras integrated in the RF coil.

9. The system of claim 7 or 8, the analyzing of the image data (116) further comprising comparing the image data with previously recorded terahertz image data (116), the threshold representing a difference of electrical conductivity of the region with respect to the previously recorded terahertz image data (116).

10. The system of claim 9, the software code, upon execution by the processor, further causing the image processing unit (110) to record the terahertz image data (116) during imaging of the subject (100) in the magnetic resonance imaging system.

11. The system of any of claims 1 - 6, the system further comprising a magnetic material detector gate to be passed by the subject (100) before performing the imaging, the terahertz camera (114) being integrated in the gate.

12. The system of any of claims 1 - 6, wherein the terahertz transmitter (112), the terahertz camera (114) and the user interface (140) are comprised in a handheld device (400).

13. A method for detecting a temporal and / or spatial characteristic of increased electrical conductivity on a human subject's skin surface, using:
- a user interface (140), and
- a skin surface conductivity detection system, comprising:
- a terahertz transmitter (112),
- a terahertz camera (114), and
- an image processing unit (110) communicatively coupled to the terahertz camera (114),
the method comprising a conductivity detection procedure comprising:
- emitting terahertz waves in the subject's direction using the terahertz transmitter (112),
- recording terahertz image data (116) from a portion of the terahertz waves reflected off the subject's skin,
- receiving the image data (116) from the terahertz camera (114) using the image processing unit (110),
- analyze the image data (116) for an occurrence of a temporal and / or spatial characteristic of electrical conductivity, the analysis comprising relating the characteristic of electrical conductivity to a predefined threshold, the threshold representing a magnitude of an electric current in the human subject's body at which a thermal burn of the subject's skin surface is avoided during exposure to an RF field of a magnetic resonance imaging system (130) for imaging the subject (100), and
- send information regarding the analysis to the user interface (140).

14. A computer program product for detecting a temporal and / or spatial characteristic of increased electrical conductivity on a human subject's skin surface, the computer program product comprising a computer-readable storage medium having program instructions embodied therewith, the program instructions being executable by a processor to cause the processor to execute the method according to claim 13.
